# EUROPEAN PATENT APPLICATION

(11) **EP 3 029 021 A1**
(43) Date of publication of application: **08.06.2016**
(21) Application number: 14832740.6
(22) Date of filing: 07.07.2014
(51) Int. Cl.: C07C 67/56, B01D 15/08, B01J 20/10, B01J 20/26, B01J 20/34, C07C 69/587, G01N 30/46, G01N 30/88

(54) **METHOD FOR SEPARATING FAT-SOLUBLE MATERIAL BY SIMULATED MOVING BED CHROMATOGRAPHY, AND DEVICE FOR SAME**

(30) Priority: 31.07.2013 JP 2013159634; 31.07.2013 JP 2013159861
(71) Applicant: Bizen Chemical Co., Ltd., Okayama 709-0716 (JP)
(72) Inventor: SHIMIZU, Yoshio, Akaiwa-shi Okayama 709-0716 (JP); TABATA, Hiroshi, Akaiwa-shi Okayama 709-0716 (JP); MANKURA, Mitsumasa, Akaiwa-shi Okayama 709-0716 (JP)
(74) Representative: Carridge, Andrew Edward
(86) International application number: PCT/JP2014/003597
(87) International publication number: WO 2015/015716

(57) **Abstract**

The present invention provides a separation method and device, comprising columns with at least two types of gels selected from the group consisting of silica gel, silver nitrate-impregnated gel and polymer gel filled therein, preferably at least three silver nitrate-impregnated gel columns and at least one silica gel column, by SMB chromatography. The present invention also provides a separation method and device, comprising columns with at least two types of columns selected from the group consisting of C18 columns, C8 columns C4 columns and C1 columns, preferably at least three C18 columns and at least one C8 column and/or C4 column and/or C1 column, by SMB chromatography.

## Description

### TECHNICAL FIELD

The present invention relates to a separation and purification method by simulated moving bed chromatography using silica gel columns and a device for the same. More specifically, the present invention relates to a separation and purification method by simulated moving bed using silica gel columns and silver nitrate-impregnated gel columns and a device for the same.

### BACKGROUND ART

Unsaturated fatty acids have similar molecular weights and chemical characteristics, and thus it is difficult to separate or purify each other. Conventionally, difference in boiling points, difference in molecular weights , and difference in degrees of solubility have been mainly utilized as methods for separating a specific fatty acid from a mixture of fatty acids or monoesters. For example, distillation, molecular sieving and supercritical fluid extraction are known. Furthermore, separation methods by the number of double bonds include wintering, urea addition, silver complex formation, and solvent separation.

Furthermore, although chromatography using silver ion treatment resin (Non-Patent Document 1) or C18 filler is also known, such chromatography has both advantages and disadvantages, such as being simple but performing insufficient separation, or being able to perform high-level purification but not suited to industrial mass processing. Hereinafter, problems of techniques used so far for purifying unsaturated fatty acids and derivatives thereof will be described. Representative separation and purification methods include (1) a vacuum precision distillation method, (2) a molecular distillation method, (3) a urea addition method, (4) a silver nitrate treatment method, (5) fixed bed chromatography, and (6) simulated moving bed chromatography. Features and problems of the respective methods are as follows.
(1) Vacuum precision distillation Method
   Feature: It is a separation method utilizing difference in boiling points of respective components.
   Problem: Thermal denaturation may occur. It takes a long period of time to purify a high-purity product.
(2) Molecular Distillation Method
   Feature: It has a low thermal influence in distilling.
   Problem: It has a low separation capability.
(3) Urea Addition Method
   Feature: It utilizes a characteristic of dissolved urea to capture linear chain molecules to form adduct crystals of a hexagonal prism while crystallizing itself.
   Problem: It has low selectivity. It also has a problem regarding waste disposal due to development of urea adduct.
(4) Silver Nitrate Treatment Method
   Feature: It utilizes a characteristic of silver nitrate solution to form a complex with regard to double bond of fatty acid.
   Problem: The price of silver is not stable. The method captures foreign substances.
(5) Fixed Bed Chromatography
   Feature: It has a low thermal influence. It is capable of performing high-accuracy separation.
   Problem: It uses a large amount of eluent. It is not suited to industrial manufacture.
(6) Simulated Moving Bed Chromatography
   Feature: It uses a small amount of eluent, and thus is suited to industrialization due to its continuous operations.
   Problem: C18 filler often used for the separation of unsaturated fatty acid is costly. It can use only a single solvent, and thus is not able to perform separation gradiently.

Unlike conventional fixed bed column methods, the simulated moving bed (hereinafter, also referred to as "SMB") chromatography uses a less amount of eluent, and is capable of performing continuous operations. As a result, the simulated moving bed chromatography is often used for industrial chromatography purification. For example, p-xylene is manufactured 500,000 t/year by SMB.

In general, it becomes possible to change elution conditions over time by utilizing gradient, and as a result, separation of respective components will be improved and the time required until elution will be shortened. With the SMB chromatography, however, it is not possible to utilize gradient of changing the ratio of different solvents in a given time, as performed in single column chromatography, and thus it is only possible to use a single eluent.

As to techniques of purifying eicosapentaenoic acid ethyl ester (hereinafter, referred to as EPA ethyl), Patent Documents 1 and 2, for example, mention SMB chromatography as an industrially excellent technique of purifying unsaturated fatty acid and derivatives thereof. The SMB chromatography makes it possible to continuously elute target components , unsaturated fatty acid and a derivative thereof, by continuously supplying raw material while retaining respective fractions formed in the layer of column adsorbent in a steady state.

In separating a highly-unsaturated fatty acid and a derivative thereof, C18 silica gel is often used as a column filler. However, C18 silica gel is costly with respect to separation performance and the objective to be achieved is further improvement on the performance in industrialization.

Thus, there is a need for achieving objectives in existing methods for purifying unsaturated fatty acid and obtaining a highly-efficient method for purifying unsaturated fatty acid and a device for the same.

In recent years, unsaturated fatty acid has been receiving attention as essential fatty acid in the field of supplements. In addition, EPA ethyl, in particular, has been approved as a drug for treating arteriosclerosis obliterans and hyperlipidemia, as a switch OTC. As a result, the market for EPA ethyl of a medicinal drug grade is expanding.

### [Prior Art Documents]

### [Patent Documents]

Patent Document 1: Japanese National Phase PCT Laid-open Publication No. 2013-516398
Patent Document 2: Japanese Laid-Open Publication No. 8-218091

### [Non-Patent Documents]

Non-Patent Document 1: Nobuyuki OTAKI: The Chemical Times, No. 3 (Volume 213) (2009)

### SUMMARY OF INVENTION

### [Problem to be solved by the Invention]

An objective to be achieved is to solve problems in existing methods for purifying unsaturated fatty acid, thereby providing a highly-efficient method for unsaturated fatty acid and a device for the same.

Furthermore, in separatinghighly-unsaturatedfatty acid and a derivative thereof, C18 column or ODS silica gel is often used as a column filler. However, such a column filler is costly with respect to separation performance. Accordingly, other objectives are to provide a relatively-inexpensive method for purifying unsaturated fatty acid and a device for the same as well as to improve separation performance thereof.

The present invention provides a technique of purifying highly-unsaturated fatty acid and a derivative thereof from animal and plant as well as microorganism fat, such as fish oil, extract oil from algae and extract oil from genetically modified plant; and in particular, provides a method for manufacturing a product of a medicinal drug grade at an industrially low cost.

### [Means for Solving the Problem]

In the present invention, the above objectives have been achieved by using a plurality of types of columns simultaneously in SMB chromatography.

In one aspect of the present invention utilizing normal phase columns, for example, the present invention uses a plurality of normal phase columns (e.g., silica gel column or polymer gel column) and one or more columns including a silver nitrate-impregnated carrier in SMB chromatography. As to eluents, a normal phase system (n-hexane), for example, can be used without limitation thereto.

In another aspect of the present invention utilizing reversed phase columns, the present invention uses four or more silica gel columns that use C18, C8, C4 and/or C1 columns in SMB chromatography. As to eluents, a normal phase system (n-hexane), for example, can be used without limitation thereto.

In one aspect of the present invention utilizing normal phase columns, the present invention provides, for example, the following:
(Item A1)
   A method for separating a fat-soluble substance by SMB chromatography,
   wherein a device for performing separation by the SMB chromatography comprises:
   (1) at least four columns including a normal phase column with a first carrier filled therein and a normal phase column with a second carrier different from the first carrier, filled therein; and
   (2) the same number of circulation lines as the number of the columns, for connecting the columns,
   wherein the method comprises:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item A2)
   A method for separating a fat-soluble substance by SMB chromatography,
   wherein a device for performing separation by the SMB chromatography comprises:
   (1) at least four columns including a normal phase column with a first carrier filled therein and a normal phase column with a second carrier different from the first carrier, filled therein, wherein the first carrier and the second carrier are selected from the group consisting of silica gel, silver nitrate-impregnated gel and polymer gel; and
   (2) the same number of circulation lines as the number of the columns, for connecting the columns,
   wherein the method comprises:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item A3)
   A method for separating a fat-soluble substance by SMB chromatography,
   wherein a device for performing separation by the SMB chromatography comprises:
   (1) at least four columns including at least one column with silver nitrate-impregnated gel filled therein as a carrier and at least one column with silica gel filled therein as a carrier; and
   (2) the same number of circulation lines as the number of the columns, for connecting the columns,
   wherein the method comprises:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item A4)
   The method according to Item A3, wherein the device for performing separation by SMB chromatography comprises: at least two columns with silver nitrate-impregnated gel filled therein as a carrier; and at least two columns with silica gel filled therein as a carrier.
(Item A5)
   The method according to Item A5, wherein the device for performing separation by SMB chromatography comprises: at least one column with silver nitrate-impregnated gel filled therein as a carrier; and at least three columns with silica gel filled therein as a carrier.
(Item A6)
   The method according to any one of Items A1 to A3, wherein the sample is a distilled sample.
(Item A7)
   The method according to any one of Items A1 to A3, wherein the fat-soluble substance is a fatty acid or a fatty acid derivative.
(Item A8)
   The method according to any one of Items A1 to A3, wherein the fat-soluble substance is selected from the group consisting of α-linolenic acid, stearidonic acid, docosapentaenoic acid, eicosapentaenoic acid, docosahexaenoic acid and lower alcohol ester thereof.
(Item A9)
   A device for separating a fat-soluble substance by SMB chromatography, comprising:
   (1) at least four columns including a normal phase column with a first carrier filled therein and a normal phase column with a second carrier different from the first carrier, filled therein; and
   (2) the same number of circulation lines as the number of the columns, for connecting the columns,
   wherein the device separates the fat-soluble substance by a method comprising:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item A10)
   A device for separating a fat-soluble substance by SMB chromatography, comprising:
   (1) at least four columns including a normal phase column with a first carrier filled therein and a normal phase column with a second carrier different from the first carrier, filled therein, wherein the first carrier and the second carrier are selected from the group consisting of silica gel, silver nitrate-impregnated gel and polymer gel; and
   (2) the same number of circulation lines as the number of the columns, for connecting the columns,
   wherein the device separates the fat-soluble substance by a method comprising:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item A11)
   A device for separating a fat-soluble substance by SMB chromatography, comprising:
   (1) at least four columns including at least one column with silver nitrate-impregnated gel filled therein as a carrier and at least one column with silica gel filled therein as a carrier; and
   (2) the same number of circulation lines as the number of the columns, for connecting the columns,
   wherein the device separates the fat-soluble substance by a method comprising:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item A12)
   The device according to Item A11, wherein the device comprises: at least two columns with silver nitrate-impregnated gel filled therein as a carrier; and at least two columns with silica gel filled therein as a carrier.
(Item A13)
   The device according to Item A11, wherein the device comprises: at least one column with silver nitrate-impregnated gel filled therein as a carrier; and at least three columns with silica gel filled therein as a carrier.

In another aspect according to the present invention utilizing reversed phase columns, the present invention provides, for example, the following:
(Item B1)
   A method for separating a fat-soluble substance by SMB chromatography,
   wherein a device for performing separation by the SMB chromatography comprises:
   (1) at least three C18 columns;
   (2) at least one column selected from the group consisting of a C8 column, a C4 column and a C1 column; and
   (3) the same number of circulation lines as the number of the columns, for connecting the columns,
   wherein the method comprises:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item B2)
   The method according to Item B1, wherein the sample is a distilled sample.
(Item B3)
   The method according to Item B1, wherein the fat-soluble substance is a fatty acid or a fatty acid derivative.
(Item B4)
   The method according to Item B1, wherein the fat-soluble substance is selected from the group consisting of α-linolenic acid, stearidonic acid, docosapentaenoic acid, eicosapentaenoic acid, docosahexaenoic acid and lower alcohol ester thereof.
(Item B5)
   A method for separating a fat-soluble substance by SMB chromatography,
   wherein a device for performing separation by the SMB chromatography comprises:
   (1) at least two types of at least four columns selected from the group consisting of a C18 column, a C8 column, a C4 column, and a C1 column; and
   (2) the same number of circulation lines as the number of the columns, for connecting the columns,
   wherein the method comprises:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item B6)
   The method according to Item B5, wherein the sample is a distilled sample.
(Item B7)
   The method according to Item B5, wherein the fat-soluble substance is a fatty acid or a fatty acid derivative.
(Item B8)
   The method according to Item B5, wherein the fat-soluble substance is selected from the group consisting of α-linolenic acid, stearidonic acid, docosapentaenoic acid, eicosapentaenoic acid, docosahexaenoic acid and lower alcohol ester thereof.
(Item B9)
   A device for separating a fat-soluble substance by SMB chromatography, comprising:
   (1) at least three C18 columns
   (2) at least one column selected from the group consisting of a C8 column, a C4 column and a C1 column; and
   (3) the same number of circulation lines as the number of the columns, for connecting the columns,
   wherein the device separates the fat-soluble substance by a method comprising:
   (a) supplying an eluent to a first circulation line;
   (b) supplying a raw material to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item B10)
   A device for separating a fat-soluble substance by SMB chromatography, comprising:
   (1) at least two types of at least four columns selected from the group consisting of a C18 column, a C8 column, a C4 column, and a C1 column; and
   (2) the same number of circulation lines as the number of the columns, for connecting the columns,
   wherein the device separates the fat-soluble substance by a method comprising:
   (a) supplying an eluent to a first circulation line;
   (b) supplying a raw material to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item B11)
   A method for separating a fat-soluble substance by SMB chromatography,
   wherein a device for performing separation by the SMB chromatography comprises:
   (1) at least two types of at least four reversed phase columns; and
   (2) the same number of circulation lines as the number of the columns, for connecting the columns,
   wherein the method comprises:
   (a) supplying an eluent to a first circulation line;
   (b) supplying a sample to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.
(Item B12)
   A device for separating a fat-soluble substance by SMB chromatography, comprising:
   (1) at least two types of at least four reversed phase columns; and
   (2) the same number of circulation lines as the number of the columns, for connecting the columns,
   wherein the device separates the fat-soluble substance by a method comprising:
   (a) supplying an eluent to a first circulation line;
   (b) supplying a raw material to a third circulation line;
   (c) eluting a separation solution from a second circulation line and a fourth circulation line; and
   (d) switching the first circulation line, the second circulation line, the third circulation line and the fourth circulation line,
   wherein the first to fourth circulation lines are arranged in the order of the first circulation line, the second circulation line, the third circulation line and the fourth circulation line from upstream to downstream.

### [Effect of the Invention]

According to the present invention, a highly-efficient method for purifying unsaturated fatty acid and a device for the same are provided.

### BRIEF DESCRIPTION OF THE DRAWINGS

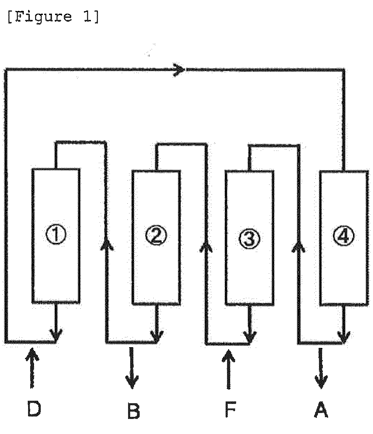

Figure 1 is a schematic view of SMB chromatography.

### EMBODIMENTS FOR CARRYING OUT THE INVENTION

Hereinafter, the present invention will be described. Throughout the present specification, unless specifically referred to, an expression in a singular form is to be understood to encompass the concept of its plurality form. Furthermore, terms usedherein , unless specifically referred to, are to be understood to be used in the meaning usually used in the art. Therefore, unless defined otherwise, all technical terms and scientific terms herein have the same meaning as generally recognized by those skilled in the art. In case of contradiction, the present specification (including the definition) governs.

### (Definition of Term)

Hereinafter, definitions of terms particularly used herein will be listed.

The term, "SMB chromatography" , as used herein refers to a separation method utilizing the principle of chromatography, which refers to a moving bed, in which a plurality of unit packed beds, with an adsorbent having different selective adsorption capabilities to a specific component and another specific component in a raw material filled therein, are connected in series and in which the unit packed bed in the downmost-stream part is connected with the packed bed in the uppermost-stream part, thereby forming an endless circulating system.

The term, "supply port of raw material", as used herein refers to a supply port of raw material provided for SMB chromatography. The term, "component eluting position", as used herein refers to an outlet port from a column of a solution provided for SMB chromatography.

The term, "upstream" , as used herein refers to a side into which an eluent flows, whereas the term, "downstream", refers to a side from which an eluent flows out.

The term, "circulation line", as used herein refers to a portion for connecting adjacent columns. The circulation line connects the component eluting position of the upstream and the supply port of raw material of the downstream, or connects the component eluting position of the downmost stream and the supply port of raw material of the uppermost stream.

The term, "switching circulation lines", as used herein refers to, in a series of columns adjacent with one another in the order of column A - column B - column C - column D from the upstream to downstream, switching the circulation line for connecting the outlet port of column B and the inlet port of column C with either (i) the circulation line for connecting the outlet port of column A and the inlet port of column B or (ii) the circulation line for connecting the outlet port of column C and the inlet port of column D.

The term, "normal phase column", as usedherein refers to a column used in a separation system in which the polarity of a stationary phase is higher than the polarity of a moving phase. Representative normal phase columns include, without limitation, silica gel column and polymer gel column.

The term, "silica gel column", as used herein refers to a chromatography column with silica gel functioning as a carrier. Silica gel columns are inexpensive and use solvents with low polarity for eluents. Thus, in the case of fish oil as a raw material, when an eluent is retrieved and repeatedly used, substances with high polarity, which is a cause of smell coming from fish oil, will not be taken in upon retrieving. As a result, there will be little deterioration in the quality as an eluent, which allows for the use over a prolonged period of time.

The term, "silver nitrate-impregnated gel", as used herein is interchangeably used with the term, "silver nitrate-impregnated carrier", and refers to a gel to which silver nitrate is adsorbed. The silver nitrate-impregnated gel is typically silica gel in which silver nitrate is impregnated. The carrier of silver nitrate-impregnated gel used in the present invention includes, without limitation, silica gel and polymer. In silver nitrate-impregnated gel, supported silver ions form a complex with molecules having double bond, thus being retained in the gel. The strength for the retaining is in accordance with the number of double bond, and thus the gel is effective in adsorbing unsaturated fatty acid or separating and purifying unsaturated fatty acid by chromatography.

In one aspect, SMB chromatography used in the present invention preferably includes one or more silver nitrate-impregnated gels and one or more silica gel columns. Preferable ratios of combination of the gel and column include silver nitrate-impregnated gel column : silica gel column = 3:1-12.

The term, "reversed phase column", as used herein refers to a column used in a separation system in which the polarity of a moving phase is higher than the polarity of a stationary phase. Representative reversed phase columns include, without limitation, a reversed silica gel column.

Theterm, "reversedsilicagelcolumn", asusedherein refers to a chromatography column with a reversed silica gel as a carrier. Representative reversed silica gel columns include, without limitation, C18 column, C8 column, C4 column, and C1 column. Reversed silica gel columns can be applied to separation of a broad range of compounds from fat-soluble to ionic substances, and they are currently the most broadly used means.

The term, "C18 column", as used herein refers to a chromatography column with which a chemically bonded-type porous and spherical silica gel, the surface of which is modified with an octadecylsilyl group, is used as a carrier. The subject column is also simply referred to as ODS column.

The term, "C8 column", as used herein refers to a chromatography column with which a chemically bonded-type porous and spherical silica gel, the surface of which is modified with an octyl group, is used as a carrier.

The term, "C4 column", as used herein refers to a chromatography column with which a chemically bonded-type porous and spherical silica gel, the surface of which is modified with a butyl group, is used as a carrier.

The term, "C1 column", as used herein refers to a chromatography column with which a chemically bonded-type porous and spherical silica gel, the surface of which is modified with a trimethyl group, is used as a carrier.

In another aspect, the SMB chromatography used in the present invention includes four or more columns. The SMB chromatography according to the present invention preferably comprises three or more C18 columns, and one or more C8 columns and/or C4 columns and/or C1 columns. Preferable ratios of the combination of such columns include C18 columns : C8 columns and/or C4 columns and/or C1 columns = 3:1-12.

For solvents used for raw material provided for the SMB chromatography according to the present invention, liquid of any degree of purity may be used so long as the liquid can be provided for column chromatography. For example, with regard to liquid extracted from raw material, liquid in which a target fatty acid is partially condensed using a method selected from the group consisting of vacuum precision distillation, filtration, urea treatment, silver nitrate treatment and fixed bed chromatography, can be used as a raw material. The raw material to be used is desirably free fatty acid or lower alcohol ester such as ethanol or methanol. In the case the raw material is in a solid form, it is dissolved with a solvent, such as methanol, ethanol or hexane, to use.

As used herein, "vacuum precision distillation" is such that: in the case of EPA, the boiling points of components whose number of carbon is 20, including EPA, are at substantially mid-boiling points among fish oil fatty acids ; in the case of batch-type, a single tower-type distillation device is required; and in the case of continuous distillation, a two tower-type device or four tower-type device is required. In the case of two tower-type, vacuum precision distillation refers to distilling C19 and other components with lower carbon numbers (initial distilling) and sending the remaining to the second tower to fractionate the C20 component (main distilling).

As used herein, "filtration" refers to a method of decoloration of raw material or removal of peroxide using a filter and/or adsorbent. Typically, it refers to a method of mixing and stirring raw material and an adsorbent in a nitrogen atmosphere and removing the adsorbent by filter press. In the method according to the present invention, preferable adsorbents include, without limitation, silica gel, activated white earth, activated charcoal, and zeolite.

As used herein, "urea treatment" refers to a method of mixing and cooling rawmaterial and a ureamethanol solution, causing the mixture to form a urea adduct in which saturated fatty acid and mono-unsaturated fatty acid are taken, followed by filtering. Typically, the urea treatment refers to a method of extraction from a urea adduct using n-hexane, treatment with silica gel, and distillation of n-hexane to obtain a target unsaturated fatty acid.

As used herein, "silver nitrate treatment" refers to the following: in the case of highly-unsaturated fatty acid ethyl, a method of stirring raw material and a silver nitrate solution, performing, for example, n-hexane-extracting on unreacted ester, diluting or heating an aqueous layer, and again performing n-hexane-extracting on free ester to condense a target highly-unsaturated fatty acid ethyl.

As used herein, "fixed bed chromatography" refers to a method of filling a column with a filler, and allowing raw material dissolved in an eluent to pass through the column to retrieve a fraction including a target component, followed by condensing. In the method according to the present invention, preferable fillers include, without limitation, silica gel, reversed phase silica gel, and silver nitrate-impregnated silica gel.

The term, "eluent", as used herein refers to a solution supplied through an eluent supply port D. Those skilled in the art can easily determine what eluent to use in accordance with gel to be used and a substance as a target for separation. As for eluents that can be used when a fat-soluble substance is separated using a silica gel column, solvents with low polarity are generally used, which include, without limitation, n-hexane, methylene chloride, benzene, cyclohexane and toluene.

The term, "fat-soluble substance", as used herein refers to compounds that dissolve in an organic solvent, such as n-hexane or ethanol. Such a fat-soluble substance includes, without limitation, fatty acid or a derivative thereof. The "fat-soluble substance" according to the present invention includes, without limitation, for example, α-linolenic acid, stearidonic acid, docosapentaenoic acid, eicosapentaenoic acid, docosahexaenoic acid and other ω3 system fatty acids or lower alcohol ester of methanol or ethanol thereof as well as γ-linolenic acid, arachidonic acid or other ω6 system fatty acids or lower alcohol ester of methanol or ethanol thereof.

The term, "loweralcoholester", as usedherein refers to ester of fatty acid or a derivative thereof and lower alcohol. The term, "lower alcohol", as used herein refers to any alcohol of linear or branched chains with the number of carbons of 10 or less.

### (SMB Chromatography)

In SMB chromatography, raw material and an eluent are supplied to an endless circulating system, and an X component moving within a column (unit packed bed) at a faster rate (i.e., weak affinity component) and a Y component moving within the column at a slower rate (i.e. , affinity component) are respectively eluted from different positions. Furthermore, in SMB chromatography, the raw material supplying position, eluent supplying position, X component eluting position and Y component eluting position are successively moved towards the downstream in the fluid circulating direction while the respective positions maintain a constant positional relationship, so as to artificially achieve treatment operations of successively performing raw material supply. As a result, the operation method allows distributions of the respective components in the bed to move with a substantially constant width and allows elution positions of the respective components to continue taking portions with both high degree of purity and concentration.

Exemplary SMB chromatography will be shown in Fig. **1****.** columns **1** to **4** are connected by circulation lines, and a raw material supply port **F**, an eluent supply port **D,** an affinity component eluting port **A,** and a weak affinity component eluting port **B** are provided for circulation lines among respective columns. Furthermore, valves are successively switched at regular time intervals in the direction of circulating flow (i.e., circulation lines are switched).

For example, raw material supplied from the raw material supply port **F** moves from the column **3** to the column **2** for an eluent supplied from the eluent supply port **D.** In this regard, the weak affinity component, X component, has weak affinity to columns and thus moves through columns at a faster rate. On the other hand, the affinity component, Y component, has strong affinity to columns and thus moves through columns at a slower rate. As a result, the X and Y components move at different rates, and as a result, they get separated from each other. In order to separate the X component from the Y component successively, it is necessary to provide the raw material supply port **F** at a position between the two components. While the Y component moves through columns at a rate slower than that of the X component, the Y component continues moving due to an eluent supplied from the eluent supply port **D.** Thus, it is necessary to switch valves sequentially at regular time intervals in the direction of circulating flow to maintain the raw material supply port **F** in between the X component and Y component. Specifically, after the elapse of a certain period of time in Fig. **1****,** the column **4** is moved to the position of the column **3,** the column **3** is moved to the position of the column **2,** the column **2** is moved to the position of the column **1,** and the column **1** is moved to the position of the column **4** (in other words, the circulation line connecting the column 1 with the column **2** is switched to the circulation line connecting the column **2** with the column **3,** the circulation line connecting the column **2** with the column **3** is switched to the circulation line connecting the column **3** with the column **4,** the circulation line connecting the column **3** with the column **4** is switched to the circulation line connecting the column **4** with the column **1,** the circulation line connecting the column **4** with the column **1** is switched to the circulation line connecting the column **1** with the column **2**). As a result, the raw material supply port **F** is moved in between the column **4** and column **3,** the weak affinity component eluting port **B** is moved in between the column **3** and column **2,** the eluent supply port **D** is moved in between the column **2** and column **1,** and the affinity component eluting port **A** is moved in between the column **1** and column **4.**

Although four or more columns are used in the SMB chromatography according to the present invention, the number of columns is not particularly limited. Preferably, the number of columns is, without limitation, 4, 5, 6, 7, 8, 9, 10, 11, 12, 13, 14, 15, 16, 17, 18, 19 or 20. When the number of columns is more than 4, a plurality of columns may be provided in between the raw material supply port **F** and the weak affinity component eluting port **B,** in between the weak affinity component eluting port **B** and the eluent supply port **D,** in between the eluent supply port **D** and the affinity component eluting port **A** and/or the affinity component eluting port **A** and the raw material supply port **F.**

### (Regarding Silica Gel)

Silica gel is often used as a carrier for normal phase chromatography. The normal phase chromatography is a method for causing respective components to be adsorbed to a surface of a filler while competing with molecules of an eluent and causing those with smaller polarity to be eluted and separated. Thus , such tendencies as described below are found with regard to the strength in retaining the components. Furthermore, silica gel is the least expensive among column fillers.
(1) The retaining is stronger as there are more double bonds of sample molecules.
(2) The retaining is stronger as the molecular weight of sample molecules is greater.

### (Regarding Silver Nitrate-impregnated Silica Gel)

Silver nitrate-impregnated silica gel allows a silver nitrate solution to pass through and causes the surface of the silica gel to support silver ions. A weak charge-transfer complex is formed between the silver ion and an unsaturated organic compound. Tendencies as described below are found with regard to the strength in retaining components.
(1) The retaining is stronger as the number of double bonds of sample molecules is larger.
(2) With regard to fatty acid (and ester thereof), the retaining is stronger as the double bond and carbonyl carbon are closer to each other.

### (Regarding C18 Silica Gel)

C18 silica gel often used for the separation of fatty acid ester has a structure in which an octadecylsilyl group is bound with a hydroxyl group on the surface of the silica gel and a hydrophobic molecule having a C₁₈ chain is extended from the surface. Due to the hydrophobic interaction produced between the hydrophobic molecule and each component, retention strength to fillers is changed and the component is separated. Tendencies as described below are found with regard to the strength in the retaining.
(1) The retaining is stronger as there are less double bonds of sample molecules.
(2) The retaining is stronger as the molecular weight of sample molecules is greater.
Accordingly, with regard to eicosapentaenoic acid(C20:5) and docosahexaenoic acid (C22:6) that are particularly of high utility value among highly-unsaturated fatty acids, the level of difficulty thereof is high in the separation with C18 silica gel due to the relationship of molecular weights and the number of double bond among respective components such as eicosatetraenoic acid (C20:4ω3), arachidonic acid (C20: 4ω6) and docosapentaenoic acid (C22 : 5), which are naturally-derived related substances. Thus, in the case of sufficiently separating the components in order to obtain high-purity products through conventional SMB, it is necessary to increase a filler with respect to samples or to decrease the amount of samples to be injected.

### (Regarding C8, C4 and C1 Silica Gel)

C8 and C4 silica gels are such that hydrophobic molecules of a C₈ chain and a C₄ chain are respectively bound to the silica gels, while C18 silica gel is such that hydrophobic molecules of a C₁₈ chain are bound to the silica gel. Thus, compared to C18, C8 and C4 silica gels have properties as described below:
(1) having weaker retention; and
(2) having lower stereospecificity.
As a result, separation patterns that are not found with C18 are exhibited and better separation conditions are provided depending on substances compared to C18 silica gel (e.g., quinine and lidocaine).

Hereinafter, the present invention will be described in detail through Examples or the like, but it should be understood that the present invention is not limited to such Examples.

### EXAMPLES

### I. Examples and Comparative Examples of the Present Invention Utilizing Normal Phase Columns

### (Comparative Example A1)

As a Comparative Example A1, a system using only silver nitrate-impregnated silica gel will be described.

As a starting material, fat containing 75% EPA ethyl ester was used. The concentration of the raw material was 200 g/L (n-hexane solution) and the supply amount of the rawmaterialwas 10. 5mL per 1L filler per hour (10.5mL/L-R/h). As for columns, four silver nitrate-impregnated silica gel columns were used (column size: 10 mm in diameter x 500 mm in height). In addition, n-hexane as an eluent was used with the eluent mount: 400 mL per 1L filler per hour (0.40L/L-R/h).

As a result, EPA ethyl with purity of 96.5% was obtained. As related substances, the following were included:

| | |
|---|---|
| octadecatetraenoic acid ethyl: | 0.61% |
| arachidonic acid ethyl: | 0.70% |
| icosatetraenoic acid ethyl: | 0.86% |
| other impurities: | 1.33% |

The recovery rate of EPA ethyl was 79%, and 1.9 for POV (peroxide value), 0.03 for AV (acid value), and 0.5 for AnV (anisidine value) respectively.

### (Example A1)

Silver nitrate-impregnated silica gel columns and silica gel columns were used in combination.

As a starting material, fat containing 75% EPA ethyl ester was used. The concentration of the raw material was 200 g/L (n-hexane solution) (i.e., 25 wt %) and the supply amount of the raw material was 16.4 mL per 1L filler per hour (16.4 mL/L-R/h). As for columns, two silver nitrate-impregnated silica gel columns and four silica gel columns were used (column size: 10 mm in diameter x 500 mm in height). In addition, n-hexane as an eluent was used with the eluent mount: 400 mL per 1L filler per hour (0.40 L/L-R/h).

As a result, EPA ethyl with purity of 98.5% was obtained. Contaminating related substances were as follows:

| | |
|---|---|
| octadecatetraenoic acid ethyl: | 0.30% |
| arachidonic acid ethyl: | 0.36% |
| icosatetraenoic acid ethyl: | 0.51% |
| other impurities: | 1.33% |

The recovery rate of EPA ethyl was 89%, and 2.0 for POV (peroxide value), 0.04 for AV (acid value), and 0.7 for AnV (anisidine value) respectively.

From the results described above, compared to simulated moving bed chromatography using only silver nitrate-impregnated silica gel columns, the supply amount of the raw material became processable 1.5 times or more. In addition, based on the analysis values, the field of application can be medicines, cosmetics and food. Thus, it became clear that EPA ethyl of a medicinal drug grade was manufacturable with a less filler amount of silver nitrate-impregnated silica gel compared to before.

### (Example A2)

Silver nitrate-impregnated silica gel columns and silica gel columns were used in a combination different from that of Example A1.

As a starting material, fat containing 75% EPA ethyl ester was used. The concentration of the raw material was 200 g/L (n-hexane solution) (i.e., 25 wt %) and the supply amount of the raw material was 16.4 mL per 1L filler per hour (16.4 mL/L-R/h). As for columns, two silver nitrate-impregnated silica gel columns and two silica gel columns were used (column size: 10 mm in diameter x 500 mm in height). In addition, n-hexane as an eluent was used with the eluent mount: 400 mL per 1L filler per hour (0.40 L/L-R/h).

As a result, EPA ethyl with purity of 97.1% was obtained. Contaminating related substances were as follows:

| | |
|---|---|
| octadecatetraenoic acid ethyl: | 0.60% |
| arachidonic acid ethyl: | 0.55% |
| icosatetraenoic acid ethyl: | 0.89% |
| other impurities: | 0.86% |

The recovery rate of EPA ethyl was 78%, and 1.8 for POV (peroxide value), 0.04 for AV (acid value), and 0.7 for AnV (anisidine value) respectively.

From the results described above, compared to simulated moving bed chromatography using only silver nitrate-impregnated silica gel columns, the supply amount of the raw material became processable 1.5 times or more.

### (Example A3)

Silver nitrate-impregnated silica gel columns and silica gel columns were used in a combination different from that of Example A1.

As a starting material, fat containing 75% EPA ethyl ester was used. The concentration of the raw material was 200 g/L (n-hexane solution) (i.e., 25 wt %) and the supply amount of the raw material was 13.5 mL per 1L filler per hour (13.5 mL/L-R/h). As for columns, one silver nitrate-impregnated silica gel column and three silica gel columns were used (column size: 10 mm in diameter x 500 mm in height). In addition, n-hexane as an eluent was used with the eluent mount: 400 mL per 1L filler per hour (0.40 L/L-R/h).

As a result, EPA ethyl with purity of 97.9% was obtained. Contaminating related substances were as follows:

| | |
|---|---|
| octadecatetraenoic acid ethyl: | 0.38% |
| arachidonic acid ethyl: | 0.40% |
| icosatetraenoic acid ethyl: | 0.67% |
| other impurities: | 0.65% |

The recovery rate of EPA ethyl was 89%, and 1.7 for POV (peroxide value), 0.03 for AV (acid value), and 0.7 for AnV (anisidine value) respectively.

From the results described above, compared to simulated moving bed chromatography using only silver nitrate-impregnated silica gel columns, the supply amount of the raw material became processable 1.3 times or more. In addition, based on the analysis values, the field of application can be medicines, cosmetics and food.

### (Combination of Silica Gel and Silver Nitrate-Impregnated Silica Gel)

In the present invention, two or more types of column fillers having different separation performance, in particular the combination of silica gel columns and silver nitrate-impregnated silica gel columns, are used in SMB chromatography, so that it has become possible to perform separation in an easy and simple manner for a plurality of substances that are difficult to separate using only a single type of column filler. Furthermore, the present invention is capable of performing separation (e.g. , purifying to reach a medicinal drug grade) with higher accuracy and a larger amount compared to the accuracy and amount of conventional SMB chromatography. In addition, in one aspect according to the present invention, it is also possible to accomplish high accuracy separation at a short period of time and at a low cost.

While not wishing to be bound by theory, with regard to the retaining strength of fatty acid ester, silver nitrate-impregnated silica gel is stronger than silica gel. Thus, while silver nitrate-impregnated silica gel has a better separation capability, it takes a long time for silver nitrate-impregnated silica gel to elute respective components as fractions. Thus, if SMB chromatography is performed with silver nitrate-impregnated silica gel for all the column fillers, the treatment capability will be a problem. Thus, silica gel was used, which was inexpensive although its separation capability was low and which took a short period of time until elution of components. As a result, an optimum combination of a separation capability and a treatment capability was made as a column filler for SMB chromatography, thereby contributing to improving purification of EPA ethyl of a medicinal drug grade, which had not been achieved with conventional silica gel only, and the treatment capability, which had been a problem with silver nitrate-impregnated silica gel only, and to reducing manufacturing cost. In the present invention that utilizes normal phase columns, polymer gel columns can also be used as normal phase columns besides silica gel columns.

### II. Examples and Comparative Examples of the Present Invention Utilizing Reversed Phase Columns

### (Comparative Example B1)

As a Comparative example B1, results obtained by using C18 silica gel only will be described hereinafter.

As a starting material, fat containing 75% EPA ethyl ester was used. The concentration of the raw material was 200 g/L (methanol solution). The supply amount of the raw material was 10.5 mL per 1L filler per hour (10.5 mL/L-R/h) . For the filler, four C18 silica gel columns were used. The column size was 10 mm in diameter x 500 mm in height. Methanol as an eluent was used with an eluent amount of 400 mL per 1L filler per hour (0.40 L/L-R/h).

The resulting purified product contained EPA ethyl of purity of 98.3%. As related substances, the following were included:

| | |
|---|---|
| octadecatetraenoic acid ethyl: | 0.31% |
| arachidonic acid ethyl: | 0.42% |
| icosatetraenoic acid ethyl: | 0.46% |
| other impurities: | 0.51% |

The recovery rate of EPA ethyl was 89%, and 1.9 for POV (peroxide value), 0.03 for AV (acid value), and 0.5 for AnV (anisidine value) respectively. From the above results, it was clarified that the subject product can be applied to the field of medicines, cosmetics and food.

### (Example B1)

Results with C18 columns and C8 columns are as described below.

The starting material was fat containing 75% EPA ethyl ester. The concentration of the raw material was 200 g/L (methanol solution), and the supply amount of the raw material was 21 mL per 1L filler per hour (21 mL/L-R/h). The columns used were three C18 silica gel columns and one C8 silica gel column (column size: 10 mm in diameter x 500 mm in height) . Methanol as an eluent was used with the eluent mount: 400 mL per 1L filler per hour (0.40 L/L-R/h).

As a result, EPA ethyl with purity of 98.5% was obtained. The following were included as related substances :

| | |
|---|---|
| octadecatetraenoic acid ethyl: | 0.22% |
| arachidonic acid ethyl: | 0.32% |
| icosatetraenoic acid ethyl: | 0.55% |
| other impurities: | 0.61% |

The recovery rate of EPA ethyl was 92%, and 2.2 for POV (peroxide value), 0.04 for AV (acid value), and 0.6 for AnV (anisidine value) respectively.

Compared to Comparative Example B1, in which only C18 columns were used, based on the above results, the supply amount of the raw material became processable about two times more. Thus, it was clarified that the present invention was able to manufacture EPA ethyl of a medicinal drug grade with a less filler amount than before.

### (Example B2)

Results obtained by using C18 columns and C8 columns with their ratio changed from that of Example B1 are as follows.

As a starting material, fat containing 75% EPA ethyl ester was used. The concentration of the raw material was 200 g/L (methanol solution), and 21 mL per 1L filler per hour (21 mL/L-R/h) was used as the supply amount of the raw material. The columns used were two C18 silica gel columns and two C8 silica gel columns (column size: 10 mm in diameter x 500 mm in height). Methanol as an eluent was used with the eluent mount: 400 mL per 1L filler per hour (0.40 L/L-R/h).

As a result, EPA ethyl with purity of 95.0% was obtained. The following were included as related substances :

| | |
|---|---|
| octadecatetraenoic acid ethyl: | 0.60% |
| arachidonic acid ethyl: | 0.84% |
| icosatetraenoic acid ethyl: | 1.37% |
| other impurities: | 2.19% |

The recovery rate of EPA ethyl was 80%, and 2.0 for POV (peroxide value), 0.02 for AV (acid value), and 0.7 for AnV (anisidine value) respectively.

From the above results, compared to Comparative Example B1, the purity was decreased by 3.3%, while the supply amount of the raw material became processable about two times more.

### (Example B3)

Results using C18 columns and C4 columns are as follows.

As a starting material, fat containing 75% EPA ethyl ester was used. The concentration of the raw material was 200 g/L (methanol solution), and 21 mL per 1L filler per hour (21 mL/L-R/h) was used as the supply amount of the raw material. The columns used were three C18 silica gel columns and one C4 silica gel column (column size: 10 mm in diameter x 500 mm in height). Methanol as an eluent was used with the eluent mount: 400 mL per 1L filler per hour (0.40 L/L-R/h).

As a result, EPA ethyl with purity of 98.2% was obtained. The following were included as related substances :

| | |
|---|---|
| octadecatetraenoic acid ethyl: | 0.30% |
| arachidonic acid ethyl: | 0.29% |
| icosatetraenoic acid ethyl: | 0.44% |
| other impurities: | 0.77% |

The recovery rate of EPA ethyl was 87%, and 2.1 for POV (peroxide value), 0.03 for AV (acid value), and 0.7 for AnV (anisidine value) respectively.

From the above results, compared to Comparative Example B1, the purity was decreased by 0.1%, while the supply amount of the raw material became processable about two times more. In addition, based on other data, it was clarified that the purification of a medicinal drug grade was performed.

### (Example B4)

Results obtained by using C18 columns and C4 columns with their ratio changed from that of Example B3 are as follows.

As a starting material, fat containing 75% EPA ethyl ester was used. The concentration of the raw material was 200 g/L (methanol solution), and 21 mL per 1L filler per hour (21 mL/L-R/h) was used as the supply amount of the raw material. The columns used were two C18 silica gel columns and two C4 silica gel columns (column size: 10 mm in diameter x 500 mm in height). Methanol as an eluent was used with the eluent mount: 400 mL per 1L filler per hour (0.40 L/L-R/h).

As a result, EPA ethyl with purity of 98.6% was obtained. The following were included as related substances :

| | |
|---|---|
| octadecatetraenoic acid ethyl: | 0.24% |
| arachidonic acid ethyl: | 0.21% |
| icosatetraenoic acid ethyl: | 0.36% |
| other impurities: | 0.59% |

The recovery rate of EPA ethyl was 76%, and 2.5 for POV (peroxide value), 0.03 for AV (acid value), and 0.7 for AnV (anisidine value) respectively.

From the above results, compared to Comparative Example B1, the purity was increased by 0.3%, while the supply amount of the raw material became processable about two times more. In addition, based on other data, it was clarified that the purification of a medicinal drug grade was performed.

### (Regarding Use of C18 Silica Gel and C8, C4 and C1 Silica Gel)

In the present invention, two or more types of column fillers having different separation performance, in particular the combination of C18 columns and C8 columns and/or C4 columns and/or C1 columns, are used in SMB chromatography, so that it has become possible to perform separation in an easy and simple manner for a plurality of substances that are difficult to separate using only a single type of column filler. Furthermore, the present invention is capable of performing separation (e. g. , purifying to reach a medicinal drug grade) with higher accuracy and a larger amount compared to the accuracy and amount of conventional SMB chromatography. In addition, in one aspect according to the present invention, it is also possible to accomplish high accuracy separation at a short period of time and at a low cost.

While not wishing to be bound by theory, C18 silica gel, C8 silica gel, C4 silica gel and C1 silica gel have low stereospecificity and thus the strength of component retention depends more on the difference in polarity than on molecular weights. As an example based on this fact, it is considered that the degree of separation was improved for components having the same molecular weight while having difference inpolarity, such as eicosapentaenoic acid (C20:5) and eicosatetraenoic acid (C20:4ω3). In addition, since C8, C4 and C1 silica gels have weak retention, the time from injection of components until elution thereof becomes shorter. Thus, the processing amount increases compared to that of C18 silica gel. It was considered that an optimum combination of a separation capability and a treatment capability was made as a column filler of SMB based on the above facts, thereby contributing to improvement on the treatment capability per filler compared to conventional C18 silica gel and to reduction of manufacturing cost.

As described above, the present invention is exemplified by the use of its preferred Embodiments. However, the present invention should not be interpreted solely based on the subject Embodiments. It is understood that the scope of the present invention should be interpreted solely based on the claims. It is understood that those skilled in the art are able to carry out an equivalent scope from the description of specific preferred Embodiments and based on the description of the present invention and common general knowledge. Furthermore, it is understood that any patent, any patent application and any references cited in the present specification should be incorporated by reference in the present specification in the same manner as the contents are specifically described therein.

### INDUSTRIAL APPLICABILITY

According to the present invention, a highly-efficient method for purifying unsaturated fatty acid and a device for the same are provided.

## Claims

1. A method for separating a fat-soluble substance by SMB chromatography,
wherein a device for performing separation by the SMB chromatography comprises:
(1) at least three C18 columns;
(2) at least one column selected from the group consisting of a C8 column, a C4 column and a C1 column; and
(3) the same number of circulation lines as the number of the columns, for connecting the columns,
wherein the method comprises:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

2. The method according to claim 1, wherein the sample is a distilled sample.

3. The method according to claim 1, wherein the fat-soluble substance is a fatty acid or a fatty acid derivative.

4. The method according to claim 1, wherein the fat-soluble substance is selected from the group consisting of α-linolenic acid, stearidonic acid, docosapentaenoic acid, eicosapentaenoic acid, docosahexaenoic acid and lower alcohol ester thereof.

5. A method for separating a fat-soluble substance by SMB chromatography,
wherein a device for performing separation by the SMB chromatography comprises:
(1) at least two types of at least four columns selected from the group consisting of a C18 column, a C8 column, a C4 column, and a C1 column; and
(2) the same number of circulation lines as the number of the columns, for connecting the columns,
wherein the method comprises:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

6. The method according to claim 5, wherein the sample is a distilled sample.

7. The method according to claim 5, wherein the fat-soluble substance is a fatty acid or a fatty acid derivative.

8. The method according to claim 1, wherein the fat-soluble substance is selected from the group consisting of α-linolenic acid, stearidonic acid, docosapentaenoic acid, eicosapentaenoic acid, docosahexaenoic acid and lower alcohol ester thereof.

9. A device for separating a fat-soluble substance by SMB chromatography, comprising:
(1) at least three C18 columns
(2) at least one column selected from the group consisting of a C8 column, a C4 column and a C1 column; and
(3) the same number of circulation lines as the number of the columns, for connecting the columns,
wherein the device separates the fat-soluble substance by a method comprising:
(a) supplying an eluent to a first circulation line;
(b) supplying a raw material to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

10. A device for separating a fat-soluble substance by SMB chromatography, comprising:
(1) at least two types of at least four columns selected from the group consisting of a C18 column, a C8 column, a C4 column, and a C1 column; and
(2) the same number of circulation lines as the number of the columns, for connecting the columns,
wherein the device separates the fat-soluble substance by a method comprising:
(a) supplying an eluent to a first circulation line;
(b) supplying a raw material to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

11. A method for separating a fat-soluble substance by SMB chromatography,
wherein a device for performing separation by the SMB chromatography comprises:
(1) at least two types of at least four reversed phase columns ; and
(2) the same number of circulation lines as the number of the columns, for connecting the columns,
wherein the method comprises:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

12. A device for separating a fat-soluble substance by SMB chromatography, comprising:
(1) at least two types of at least four reversed phase columns ; and
(2) the same number of circulation lines as the number of the columns, for connecting the columns,
wherein the device separates the fat-soluble substance by a method comprising:
(a) supplying an eluent to a first circulation line;
(b) supplying a raw material to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

13. A method for separating a fat-soluble substance by SMB chromatography,
wherein a device for performing separation by the SMB chromatography comprises:
(1) at least four columns including a normal phase column with a first carrier filled therein and a normal phase column with a second carrier different from the first carrier, filled therein; and
(2) the same number of circulation lines as the number of the columns, for connecting the columns,
wherein the method comprises:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

14. A method for separating a fat-soluble substance by SMB chromatography,
wherein a device for performing separation by the SMB chromatography comprises:
(1) at least four columns including a normal phase column with a first carrier filled therein and a normal phase column with a second carrier different from the first carrier, filled therein, wherein the first carrier and the second carrier are selected from the group consisting of silica gel, silver nitrate-impregnated gel and polymer gel; and
(2) the same number of circulation lines as the number of the columns, for connecting the columns,
wherein the method comprises:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

15. A method for separating a fat-soluble substance by SMB chromatography,
wherein a device for performing separation by the SMB chromatography comprises:
(1) at least four columns including at least one column with silver nitrate-impregnated gel filled therein as a carrier and at least one column with silica gel filled therein as a carrier; and
(2) the same number of circulation lines as the number of the columns, for connecting the columns,
wherein the method comprises:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

16. The method according to claim 15, wherein the device for performing separation by SMB chromatography comprises: at least two columns with silver nitrate-impregnated gel filled therein as a carrier; and at least two columns with silica gel filled therein as a carrier.

17. The method according to claim 15, wherein the device for performing separation by SMB chromatography comprises: at least one column with silver nitrate-impregnated gel filled therein as a carrier; and at least three columns with silica gel filled therein as a carrier.

18. The method according to any one of claims 13 to 15 , wherein the sample is a distilled sample.

19. The method according to any one of claims 13 to 15, wherein the fat-soluble substance is a fatty acid or a fatty acid derivative.

20. The method according to any one of claims 13 to 15, wherein the fat-soluble substance is selected from the group consisting of α-linolenic acid, stearidonic acid, docosapentaenoic acid, eicosapentaenoic acid, docosahexaenoic acid and lower alcohol ester thereof.

21. A device for separating a fat-soluble substance by SMB chromatography, comprising:
(1) at least four columns including a normal phase column with a first carrier filled therein and a normal phase column with a second carrier different from the first carrier, filled therein; and
(2) the same number of circulation lines as the number of the columns, for connecting the columns,
wherein the device separates the fat-soluble substance by a method comprising:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

22. A device for separating a fat-soluble substance by SMB chromatography, comprising:
(1) at least four columns including a normal phase column with a first carrier filled therein and a normal phase column with a second carrier different from the first carrier, filled therein, wherein the first carrier and the second carrier are selected from the group consisting of silica gel, silver nitrate-impregnated gel and polymer gel; and
(2) the same number of circulation lines as the number of the columns, for connecting the columns,
wherein the device separates the fat-soluble substance by a method comprising:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

23. A device for separating a fat-soluble substance by SMB chromatography, comprising:
(1) at least four columns including at least one column with silver nitrate-impregnated gel filled therein as a carrier and at least one column with silica gel filled therein as a carrier; and
(2) the same number of circulation lines as the number of the columns, for connecting the columns,
wherein the device separates the fat-soluble substance by a method comprising:
(a) supplying an eluent to a first circulation line;
(b) supplying a sample to a third circulation line;
(c) eluting a separation solution from a second circulation line and a fourth circulation line; and
(d) switching the first circulation line, the second circulation line, the third circulation line and the fourth circulation line,
wherein the first to fourth circulation lines are arranged in the order of the first circulation line, the second circulation line, the third circulation line and the fourth circulation line from upstream to downstream.

24. The device according to claim 23, wherein the device comprises: at least two columns with silver nitrate-impregnated gel filled therein as a carrier; and at least two columns with silica gel filled therein as a carrier.

25. The device according to claim 23, wherein the device comprises: at least one column with silver nitrate-impregnated gel filled therein as a carrier; and at least three columns with silica gel filled therein as a carrier.
